# EUROPEAN PATENT APPLICATION

(11) **EP 4 644 364 A1**
(43) Date of publication of application: **05.11.2025**
(21) Application number: 24305707.2
(22) Date of filing: 03.05.2024
(51) Int. Cl.: C07C 253/26, C07C 255/08

(54) **OPEN LOOP FOR FEEDSTOCK DELIVERY AND REFRIGERATION**

(71) Applicant: T.EN Process Technology, Inc., Houston, TX 77079 (US)
(72) Inventor: Roux, Jean-Francois, Houston, 77079 (US)
(74) Representative: Edson, Russell Gregory

(57) **Abstract**

A chemical manufacturing process may include an open loop that provides both feedstock delivery and refrigeration. For example, the process may include conveying a hydrocarbon feedstock (e.g., propane or propylene) in a liquid phase from a feedstock accumulator to a shell side of a heat exchanger and vaporizing the feedstock in the heat exchanger. Then, the method may include collecting the feedstock in a vapor phase from the heat exchanger in an economizer; compressing and condensing a first portion of the feedstock in the vapor phase from the economizer in a compressor; and recycling the feedstock in the liquid phase from the compressor to the feedstock accumulator, which closes the refrigeration loop. As the open part of the loop to provide feedstock to the reactor, the method may further include conveying a second portion of the feedstock in the vapor phase from the economizer to a reactor.

## Description

### Technical Field

The present disclosure relates generally to open loops that provide both feedstock delivery and refrigeration. More specifically, but not by way of limitation, this disclosure relates to the use of an open loop for handling propylene and/or propane feedstock that allows at least a portion of the feedstock delivered to a reactor or other system component to first provide refrigeration for one or more system components.

### Background

Propylene ammoxidation is currently the primary process, if not the sole process, used for acrylonitrile production. Further, the Sohio process (using fluidized bed reactor) is used in propylene ammoxidation. The Sohio process includes a closed refrigerant loop using refrigerated water (e.g., a mixture of glycol and brine). The refrigerant cycle uses propylene, also in a closed loop, to cool the refrigerated water. Also, the Sohio process starts with high pressure liquid feedstocks of propylene and ammonia that are letdown in pressure and heated before introduction to the reactor. The lean water from the absorber is typically passed through a heat exchanger against the propylene feedstock or ammonia feedstock, each being vaporized, to cool the lean water and heat the feedstocks. This yields a complex system that is sensitive to propylene upsets and has inefficiencies, especially in the two closed loop refrigeration systems. Similarly complex systems with the similar disadvantages are present in other chemical manufacturing systems and processes.

### Summary

One or more embodiments include a chemical manufacturing process comprising: conveying a hydrocarbon feedstock in a liquid phase from a feedstock accumulator to a shell side of at least one heat exchanger in parallel, wherein the hydrocarbon feedstock comprises propane or propylene; vaporizing the hydrocarbon feedstock in the shell side of the at least one heat exchanger; collecting the hydrocarbon feedstock in a vapor phase from the at least one heat exchanger in an economizer that contains the hydrocarbon feedstock in both the liquid phase and the vapor phase; compressing and condensing a first portion of the feedstock in the vapor phase from the economizer in a compressor; recycling the feedstock in the liquid phase from the compressor to the feedstock accumulator; and conveying a second portion of the hydrocarbon feedstock in the vapor phase from the economizer to a reactor.

One or more embodiments include the process of any previous paragraph, wherein the process further comprises: letting down a pressure of the hydrocarbon feedstock in a liquid phase before introduction to the shell side of the at least one heat exchanger.

One or more embodiments include the process of any previous paragraph, wherein the shell side of the at least one heat exchanger operates at a temperature ranging from 10 °C to 20 °C and at a pressure within 200 kPa of a vapor pressure of the hydrocarbon feedstock.

One or more embodiments include the process of any previous paragraph, wherein the process further comprises: controlling a level of the feedstock in the liquid phase in the economizer with the feedstock in the liquid phase from the feedstock accumulator.

One or more embodiments include the process of any previous paragraph, wherein the at least one heat exchanger is at least one first heat exchanger, and wherein the method further comprises: conveying the feedstock in the liquid phase from the economizer to a shell side of at least one second heat exchanger in parallel; vaporizing the feedstock in a shell side of the at least one second heat exchanger; collecting the feedstock in the vapor phase from the at least one second heat exchanger in a vessel; compressing and condensing the feedstock in the vapor phase from the vessel in a first stage of the compressor; and wherein the compressing and condensing of the first portion of the feedstock in the vapor phase from the economizer is in a second stage of the compressor.

One or more embodiments include the process of any previous paragraph, wherein the process further comprises: letting down a pressure of the hydrocarbon feedstock in a liquid phase before introduction to the shell side of the at least one second heat exchanger.

One or more embodiments include the process of any previous paragraph, wherein the shell side of the at least one heat second exchanger operates at a temperature ranging from -2 °C to 8 °C and at a pressure within 200 kPa of a vapor pressure of the hydrocarbon feedstock.

One or more embodiments include the process of any previous paragraph, wherein the at least one heat exchanger is at least one first heat exchanger, and wherein the method further comprises: passing the feedstock in the liquid phase from the compressor through a tube side of at least one second heat exchanger in parallel before introduction to the feedstock accumulator.

One or more embodiments include the process of any previous paragraph, wherein the reactor is an ammoxidation reactor, and wherein the method further comprises: passing an ammonia feedstock through a shell side of one or more of the at least one second heat exchanger.

One or more embodiments include the process of any previous paragraph, wherein the ammonia feedstock after passing through the shell side of the one or more of the at least one second heat exchanger is at a temperature ranging from 19 °C to 29 °C.

One or more embodiments include the process of any previous paragraph, wherein the process further comprises: heating the ammonia feedstock downstream of the one or more of the at least one second heat exchanger to a temperature ranging from 60 °C to 70 °C before introduction to the reactor.

One or more embodiments include the process of any previous paragraph, wherein the process further comprises: heating the second portion of the hydrocarbon feedstock from the economizer before introduction to the reactor.

One or more embodiments include a chemical manufacturing system comprising: a reactor; at least one heat exchanger in parallel; a feedstock accumulator; an economizer; and a compressor, wherein the chemical manufacturing system includes: an open refrigerant cycle flow path comprising, in a flow direction of a hydrocarbon feedstock: the feedstock accumulator, a shell side of the at least one heat exchanger, the economizer, the compressor, and the feedstock accumulator, and a feedstock delivery flow path comprising, in a flow direction of the hydrocarbon feedstock: the feedstock accumulator, the shell side of the at least one heat exchanger, the economizer, and the reactor.

One or more embodiments include the chemical manufacturing system of any previous paragraph, wherein the at least one heat exchanger is at least one first heat exchanger, wherein the open refrigerant cycle flow path further comprises a shell side of at least one second heat exchanger in parallel and a vessel in series and between the economizer and a first stage of the compressor, and wherein the open refrigerant cycle flow path further comprises a second stage of the compressor immediately downstream of the economizer.

One or more embodiments include the chemical manufacturing system of any previous paragraph, wherein the open refrigerant cycle flow path further comprises at least one control valve between the economizer and the shell side of the at least one second heat exchanger.

One or more embodiments include the chemical manufacturing system of any previous paragraph, wherein the at least one heat exchanger is at least one first heat exchanger, and wherein the open refrigerant cycle flow path further comprises a tube side of at least one second heat exchanger in parallel between the compressor and the feedstock accumulator.

One or more embodiments include the chemical manufacturing system of any previous paragraph, wherein the system further comprises an ammonia feedstock flow path to the reactor that passes through a shell side of one or more of the at least one second compressor before introduction to the reactor.

One or more embodiments include the chemical manufacturing system of any previous paragraph, wherein the open refrigerant cycle flow path further comprises at least one control valve between the feedstock accumulator and the shell side of the at least one heat exchanger.

One or more embodiments include an ammoxidation process comprising: reacting propylene, ammonia, and air in a reactor to yield a raw product; contacting the raw product with water in an absorber; passing water extracted from the absorber through a tube side of at least one heat exchanger in parallel in a water cooling cycle, wherein the water cooling cycle cools the water extracted from the absorber and recycles the water extracted from the absorber back to the absorber; and passing the propylene through a shell side of the at least one heat exchanger before introducing the propylene to the reactor.

One or more embodiments include the ammoxidation process of any previous paragraph, wherein the at least one heat exchanger is at least one first heat exchanger, and wherein the method further comprises: passing the water extracted from the absorber through a tube side of at least one second heat exchanger in parallel that is downstream of the at least one first heat exchanger.

One or more embodiments include the ammoxidation process of any previous paragraph, wherein the at least one heat exchanger is at least one first heat exchanger, and wherein the method further comprises: passing the ammonia through a shell side of at least one second heat exchanger before introduction to the reactor; and passing the propylene through the tube side of the at least one second heat exchanger, wherein the at least one heat exchanger and the at least one second heat exchanger are a portion of an open refrigerant cycle through which at least a portion of the propylene passes before introduction to the reactor.

### Brief Description of the Drawings

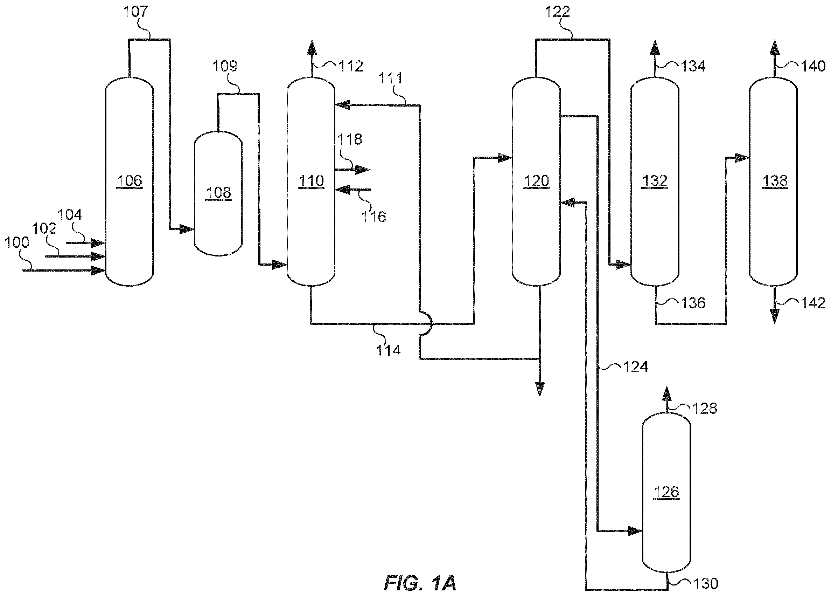

FIG. 1A illustrates a process flow diagram for synthesizing and purifying acrylonitrile within a propylene ammoxidation process, according to at least some embodiments of the present disclosure.

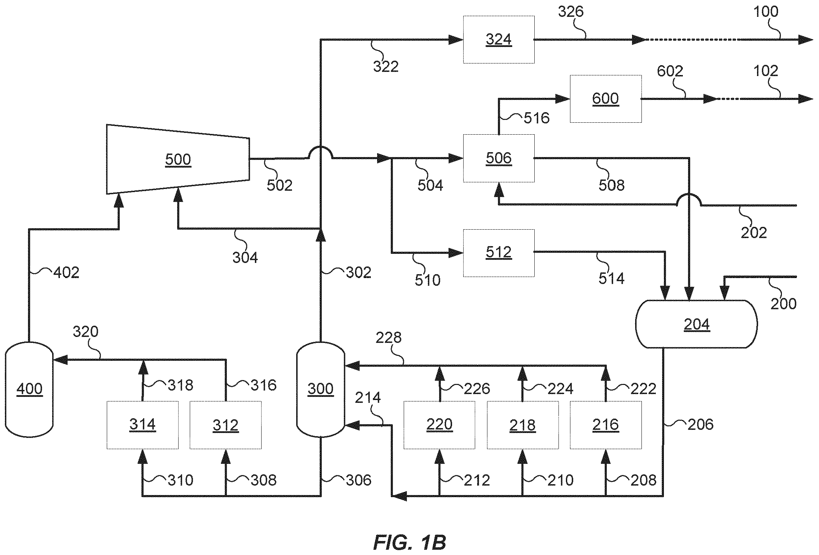

FIG. 1B illustrates a process flow diagram for an open loop feedstock delivery and refrigeration process within the propylene ammoxidation process, according to at least some embodiments of the present disclosure.

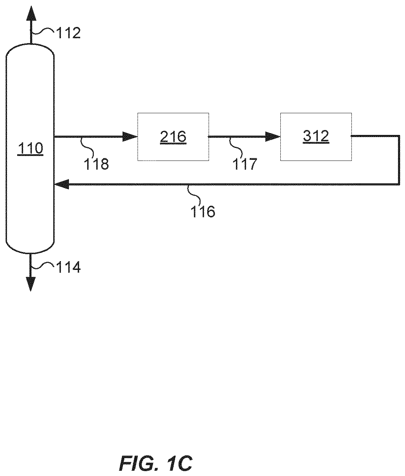

FIG. 1C illustrates a process flow diagram for a water cooling cycle associated with an absorber within the propylene ammoxidation process, according to at least some embodiments of the present disclosure.

### Detailed Description

The present disclosure integrates feedstock delivery and refrigeration using an open loop, which may be used in various chemical manufacturing systems and processes that have propane or propylene feedstocks. Examples of such chemical manufacturing processes may include propylene ammoxidation to acrylonitrile, acrylic acid production using propane, and propane dehydration to produce propylene.

The present disclosure takes advantage of the vapor pressure and temperature relationship and routes at least a portion of the propylene or propane feedstock through the heat exchangers as a coolant before sending the feedstock to the reactor. This open loop, which integrates feedstock delivery and refrigeration, has several advantages over chemical manufacturing processes and systems that use one or more closed loops for refrigeration.

For example, the open loop includes an accumulator for the propylene or propane that may be maintained at levels that mitigate, if not eliminate, the impact of upsets in propylene or propane supply on the efficiency the refrigeration and the reaction. This provides a more stable and reliable chemical manufacturing process and system.

Further, the refrigeration portion of the open loop of the present disclosure allows the propylene or propane to be both (1) the feed stream and (2) the coolant in portions of the chemical manufacturing process rather than an additional coolant for the coolant (e.g., refrigerated water). In a few instances, propylene or propane has been used for refrigeration, but the propylene or propane was a product of the manufacturing process, and not a feed as with the present disclosure. Further, where propylene or propane are used for refrigeration, the use is in a single heat exchanger. In contrast, the propylene or propane feed of the present disclosure is used in multiple locations in an order that maximizes recovery of the cold duty of the propylene or propane.

Using propylene or propane as both a feed and a refrigerant also reduces the equipment requirements and increases efficiency. For example, implementation of at least one embodiment of the present disclosure in an ammoxidation system eliminates the need for several pieces of equipment.

Additionally, with less equipment, there is less heat loss and, therefore, less power and additional duty required from supporting systems like the propylene compressor, cooling water, and low pressure steam.

These illustrative examples are given to introduce the reader to the general subject matter discussed here and are not intended to limit the scope of the disclosed concepts. The following sections describe various additional features and examples with reference to the drawings in which like numerals indicate like elements but, like the illustrative examples, should not be used to limit the present disclosure.

Generally, the open refrigerant loop includes a feedstock accumulator that receives a hydrocarbon feedstock of propylene or propane as a high pressure liquid. The feedstock accumulator may be a large drum or other vessel that can contain a sufficient volume of the hydrocarbon feedstock to mitigate an upset in the supply from impacting downstream processes.

The open refrigerant loop also includes the shell side of one or more heat exchangers in parallel and downstream of the feedstock accumulator. As used herein, one or more apparatuses in parallel encompasses one apparatus alone or two or more apparatuses in parallel. The hydrocarbon feedstock undergoes vaporization in the shell side of the one or more heat exchangers, so the shell side contains the hydrocarbon feedstock in both the liquid and vapor phases. The pressure in the shell side may be used to control the temperature according to the vaporization pressure of the hydrocarbon feedstock.

The one or more heat exchangers may be heat exchangers that are a part of or support the chemical manufacturing process where the use of the hydrocarbon feedstock in the shell side replaces a refrigerant (e.g., a mixture of glycol and brine refrigerant in propylene ammoxidation and a propylene refrigerant in propane dehydration). The tube side of heat exchangers includes a fluid to be cooled by the shell side hydrocarbon feedstock where said fluid is a part of or supports the chemical manufacturing process. One skilled in the art, with the benefit of this disclosure, will recognize appropriate locations for integrating the open refrigerant loop described herein into existing chemical manufacturing process and systems.

An economizer is also present in the open refrigerant loop and is downstream of the one or more heat exchangers. The hydrocarbon feedstock in the vapor phase produced in the one or more heat exchangers may be collected in the economizer. The economizer may contain hydrocarbon feedstock in both the liquid and vapor phases. Liquid hydrocarbon feedstock from the feedstock accumulator may be used to control the level of hydrocarbon feedstock liquid in the economizer. Suitable sensors, valves, and other equipment may be used to monitor and control the liquid hydrocarbon feedstock level by conveying liquid hydrocarbon feedstock, as needed, from the feedstock accumulator to the economizer.

The open refrigerant loop also includes a compressor downstream of the economizer for compressing and condensing hydrocarbon feedstock in the vapor phase. In a simplest configuration, said hydrocarbon feedstock vapor may be supplied directly from the economizer. However, the liquid phase from the economizer may be supplied to additional heat exchangers that produce hydrocarbon feedstock in the vapor phase in a similar manner as discussed above. The produced hydrocarbon feedstock in the vapor phase may also be collected in a vessel and conveyed to the compressor for compressing and condensing.

The hydrocarbon feedstock in the liquid form produced by the compressor is conveyed downstream (optionally with intervening processing) to the feedstock accumulator, thereby completing the open refrigerant loop.

Accordingly, an open refrigerant loop may have a flow path of the hydrocarbon feedstock that includes there along, in the direction of hydrocarbon feedstock flow: the feedstock accumulator, a shell side of the at least one heat exchanger in parallel, the economizer, the compressor, and the feedstock accumulator. The compressor is downstream of an outlet (or portion) of the economizer for hydrocarbon feedstock in the vapor phase.

In some instances, the at least one heat exchanger may be at least one first heat exchanger, and the open refrigerant cycle flow path may further include a shell side of at least one second heat exchanger in parallel and a vessel, where the shell side(s) and the vessel are in series and located along the flow path between the economizer and a first stage of the compressor. The shell side of at least one second heat exchanger is downstream of an outlet (or portion) of the economizer for hydrocarbon feedstock in the liquid phase. Further, a second stage of the compressor may be immediately downstream of the economizer.

In all of the following, a same reference designates a stream flowing in a conduit and the conduit which conveys this stream.

FIGS. 1A-1C illustrate process flow diagrams of various subprocesses within a propylene ammoxidation process like a SOHIO process. Specifically, FIG. 1A illustrates a process flow diagram for synthesizing and purifying acrylonitrile within the propylene ammoxidation process, according to at least some embodiments of the present disclosure. FIG. 1B illustrates a process flow diagram for an open loop feedstock delivery and refrigeration process within the propylene ammoxidation process, according to at least some embodiments of the present disclosure. FIG. 1C illustrates a process flow diagram for a water cooling cycle associated with an absorber within the propylene ammoxidation process, according to at least some embodiments of the present disclosure.

Referring first to FIG. 1A, the acrylonitrile synthesis and purification subprocesses can include introducing propylene 100, ammonia 102, and air 104 into a reactor 106. The various feeds can be introduced individually, as a mixture of two or more feeds, or a combination thereof (e.g., one feed introduced alone, and two feeds introduced as a mixture). The reactor 106 contains a catalyst and may be a fluidized bed reactor. The feeds react in the reactor 106 to produce nitriles. The reaction is highly exothermic. The raw product 107 includes acrylonitrile, acetonitrile, hydrogen cyanide, unreacted feeds, and inert gases (e.g., N₂, CO₂, and O₂). The raw product 107 is then cooled down in a quench tower 108 before being purified by a series of processes.

In the illustrated example, the raw product 109 from the quench tower 108 is passed through an absorber 110 where the raw product 109 is contacted with a counterflow of water 116. Part of this water can be taken from a downstream recovery column 120 bottoms (illustrated as stream 111) and/or can be fresh water (not illustrated). The water is supplied to a top part of the absorber 110 and cools down the off-gases 112 of the absorber 110. To cool down the absorber 110, side exchangers are traditionally used where stream 118 is cooled down before being reinjected into the absorber 110 at stream 116.

Further, the off-gases 112 from the absorber 110 include gaseous species like unreacted propylene, carbon monoxide, carbon dioxide, and nitrogen. The liquid that collects at the bottom (or liquid bottoms 114) is further purified. The liquid bottoms 114 may comprises acrylonitrile, acetonitrile, hydrogen cyanide, and water.

In the illustrated example, the liquid bottoms 114 is separated in an acrylonitrile recovery column 120 (e.g., a stripper column) into bottoms 121 and crude acrylonitrile 122. Generally, a side stream 124 is further processed in an acetonitrile column 126 (e.g., a distillation column) to separate the crude acetonitrile 128. The acetonitrile column bottoms 130 is sent back to the recovery column 120. Generally, the bottoms 121 includes primarily water and trace organics. These organics can be at last partially removed so that the water of the bottoms 121 can be reinjected to into the quench tower 108 and/or the absorber 110.

Generally, the crude acrylonitrile 122 includes acrylonitrile and hydrogen cyanide. The crude acrylonitrile 122 is further processed in a hydrogen cyanide column 132 (also referred to as a lights column) (e.g., a distillation column or distillation still) to remove the hydrogen cyanide 134. The remaining bottoms 136 from the hydrogen cyanide column 132 is processed in a product distillation column 138 (also referred to as an acrylonitrile column) (e.g., a distillation column) to and produce acrylonitrile 140 with high purity. Acrylonitrile bottoms 142 not meeting the desired specification (e.g., because of a high concentration of impurities) can be recycled back upstream in the process for further purification.

The reaction and purification conditions for each of these is well-known in the art. Further, modifications to the acrylonitrile synthesis and purification subprocesses are well-known in the art. For example, catalysts, conditions, and modifications are described in US Patent Nos. 4,269,667, 5,079,207, 5,629,444, and 5,770,757.

Referring now to FIG. 1B, the open loop feedstock delivery and refrigeration process within the propylene ammoxidation process includes inputs of propylene 200 and ammonia 202 and outputs propylene 100 and ammonia 102 for use as feeds to the reactor 106 of FIG. 1A. Generally, at least a portion of the propylene 200 is used in the open loop as a refrigerant for several heat exchangers (e.g., kettle exchangers 216, 218, 220, 312, 314) and as a heat supply for at least one heat exchanger (e.g., kettle exchanger 506). As discussed previously, current technologies use a separate refrigerant, typically a brine and glycol mixture, in a closed loop for a refrigerant.

The propylene 200 is introduced to a propylene accumulator 204, illustrated as a horizontal drum, and then distributed to a first set of heat exchangers (illustrated as kettle exchangers 216, 218, 220) as liquid propylene 206. The propylene accumulator 204 may be elevated in comparison to, for example, downstream heat exchangers, so that the liquid propylene 206 can flow by gravity for distribution. Elevation for the propylene accumulator 204 is preferably above (more preferably only slightly above) the location of the feed valves for each of the kettle exchangers 216, 218, 220 to avoid any flashing prior to reaching the feed valves. Optionally, a pump can be used to distribute or assist with distribution of the liquid propylene 206.

As illustrated, three portions 208, 210, 212 of the liquid propylene 206 are distributed to the kettle exchangers 216, 218, 220, respectively. A fourth portion 214 of the liquid propylene 206 is sent directly to a propylene economizer 300.

The portions 208, 210, 212 of the liquid propylene 206 are letdown using control valves (not illustrated) before introduction to a shell side of kettle exchangers 216, 218, 220, respectively. Propylene vapor 222, 224, 226 exits the shell side of kettle exchangers 216, 218, 220, respectively. In each kettle exchanger shell, the propylene liquid level is controlled using corresponding upstream control valves (not illustrated) for the propylene vapor 222, 224, 226. The present illustration shows three kettle exchangers. However, any number of heat exchangers (e.g., 1 to 6 and, typically, 1 to 4) of any suitable type may be used depending on the configuration of the propylene ammoxidation process. The propylene passing through the kettle exchanger 216 cools the lean water in the lean water cooling cycle associated with the absorber 110, which is discussed in more detail at FIG. 1C. In the illustrated example, the kettle exchangers 218 and 220 are condensers for the hydrogen cyanide column 132 and product distillation column 138, respectively.

The propylene vapor 222, 224, 226 from all kettle exchangers 216, 218, 220 is combined 228 and conveyed to a propylene economizer 300, illustrated as a vertical drum. Further, the fourth portion 214 of the propylene liquid 206 can also be sent directly to the propylene economizer 300. A control valve (not illustrated) located for the fourth portion 214 can be used to control the liquid level in the propylene economizer 300.

Propylene vapor 302 from the propylene economizer 300 is be split into two portions 304, 322. The first portion 304 of the propylene vapor 302 is conveyed to a propylene compressor 500.

The second portion 322 is conveyed to a propylene feedstock superheater 324 (e.g., a heat exchanger). The propylene feedstock superheater 324 can be a shell and tube exchanger operating against low pressure steam condensing to low pressure condensate. The resultant superheated propylene 326 is letdown using a control valve (not illustrated) before being conveyed as the propylene 100 to the reactor 106 of the propylene ammoxidation process. That is, the superheated propylene 326 is the source of the propylene 100 for the propylene ammoxidation process. The control valve for the superheated propylene 326 that reduces the pressure of the superheated propylene can also serve as a flow controller for the propylene 100 to the reactor 106.

The propylene economizer 300 may be elevated in comparison to, for example, downstream heat exchangers, so that liquid propylene 306 can flow by gravity for further distribution. Optionally, a pump can be used to distribute or assist with distribution of the liquid propylene 306. As illustrated, the liquid propylene 306 is distributed in two portions 308, 310 to the shell side of two kettle exchangers 312, 314, respectively. A control valve (not illustrated) is used to letdown the pressure for each of the two portions 308, 310 of the liquid propylene 306. The present illustration shows two kettle exchangers 312, 314. However, any number of heat exchangers (e.g., 1 to 5 and, typically, 1 to 3) of any suitable type may be used depending on the configuration of the propylene ammoxidation process. The propylene passing through the kettle exchanger 312 cools the water in the water cooling cycle associated with the absorber 110, which is discussed in more detail at FIG. 1C. In the illustrated example, the kettle exchanger 314 is cooling down the hydrogen cyanide 134.

In the shell of each kettle exchanger 312, 314, the propylene liquid level can be controlled using an upstream control valve (not illustrated) at the effluent propylene vapor 316, 318, respectively. The propylene vapor 316, 318 from all of the kettle exchangers 312, 314 is combined 320 and conveyed to the propylene compressor suction drum 400, illustrated as a vertical drum.

The propylene compressor 500 compresses propylene vapor supplied thereto in two stages. The first stage of the propylene compressor 500 receives propylene vapor 402 from the propylene compressor suction drum 400. The second stage receives propylene vapor from the first stage and propylene vapor 304 originating from the propylene economizer 300.

The propylene compressor 500 discharges compressed propylene vapor 502 that is split into two portions 504, 510. The first portion 504 is conveyed to a heat exchanger, which, in this example, is the tube side of a kettle exchanger 506. The second portion 510 is conveyed to a propylene condenser 512.

In the kettle exchanger 506, the propylene vapor condenses, and the resulting propylene liquid 508 is conveyed to the propylene accumulator 204. The kettle exchanger 506 can be positioned to allow for gravity flow to convey the propylene liquid 508 to the propylene accumulator 204. Optionally, a pump can be used to convey or assist with conveyance of the liquid propylene 508. The shell side of the kettle exchanger 506 is used to vaporize the ammonia 202, discussed in more detail below.

The second portion 510 of the propylene vapor 502 from the propylene compressor 500 is conveyed to the propylene condenser 512 (e.g., a kettle exchanger). The resultant propylene liquid 514 is then conveyed to the propylene accumulator 204. The propylene condenser 512 can be positioned to allow for gravity flow to convey the propylene liquid to the propylene accumulator 204. Optionally, a pump can be used to convey or assist with conveyance of the liquid propylene 514. The propylene condenser 512 can be operated against a cooling fluid (e.g., recirculating cooling water) that condenses the vapor into the liquid propylene 514.

Referring now to the processing of the ammonia 202, the ammonia 202 is introduced to the shell side of the kettle exchanger 506 to produce ammonia vapor 516. As discussed above, the tube side of the kettle exchanger 506 is the first portion 504 of the propylene vapor 502. The liquid level of ammonia in the kettle exchanger 506 can be controlled using a control valve (not illustrated) located upstream on the kettle exchanger 506.

The ammonia vapor 516 is conveyed from the kettle exchanger 506 to an ammonia superheater 600 (e.g., a heat exchanger). The ammonia superheater 600 can be a shell and tube exchanger operating against low pressure steam condensing to low pressure condensate. The resultant superheated ammonia 602 is conveyed to the reactor 106 and used as a feed in the propylene ammoxidation process. That is, the superheated ammonia 602 is the source of the ammonia 102 for the propylene ammoxidation process. The flowrate of ammonia 102 to the reactor 106 can, for example, be controlled by controlling the rate of vaporization in the kettle exchanger 506, which can be controlled by adjusting the flow rate of propylene vapor to the kettle exchanger 506 using a flow control valve (not illustrated) located for the first portion 504 of the propylene vapor 502.

Additionally, normal no flow (NNF) lines (not illustrated) may be included in the illustrated open loop feedstock delivery and refrigeration process. The NNF lines are typically used during startup, shutdown, and upsets to provide proper propylene flow when operating under non-steady state conditions. Examples of locations for NNF lines may include, but are not limited to, flow from the propylene compressor 500 discharge the propylene economizer 300 and flow from the propylene compressor 500 discharge to the propylene compressor suction drum 400.

Referring now to FIG. 1C, as discussed above, the absorber 110 in the propylene ammoxidation process has a water cooling cycle associated therewith. Hot water 118 that was heated in the absorber 110 is conveyed from the absorber 110 to the kettle exchanger 216 to cool the hot water 118 and produce warm water 117. Specifically, in the illustrated example, the hot water 118 is introduced to the tube side of the kettle exchanger 216, and the portion 208 of the liquid propylene 206 is introduced to the shell side of the kettle exchanger 216.

The warm water 117 is further cooled to produce cool water 116 using the kettle exchanger 312. Specifically, in the illustrated example, the warm water 117 is introduced to the tube side of the kettle exchanger 312, and the portion 308 of the liquid propylene 306 is introduced to the shell side of the kettle exchanger 312. The resultant cool water 116 is introduced back into the absorber 110.

Table 1 provides example operating temperatures and pressures for propylene and ammonia at various points of FIGS. 1A-1C.

**Table 1 - Nonlimiting Example of Operating Conditions**

| Component | Operating Temperature (°C) | Operating Pressure (kPa gauge) |
|---|---|---|
| Propylene 100 | 60-70 (e.g., 62-68 or 65) | 50-250 (e.g., 80-150 or 110) |
| Propylene accumulator 204 | Tv | 1200-1600 (e.g., 1300-1500 or 1400) |
| Propylene in kettle exchangers 216, 218, 220 | 10-20 (e.g., 12-18 or 15) | Pv (e.g., about 800±200) |
| Propylene economizer 300 | 10-20 (e.g., 12-18 or 15) | Pv (e.g., about 800±200) |
| Propylene in kettle exchangers 312, 314 | -2-8 (e.g., 0-6 or 3) | Pv (e.g., about 500±200) |
| Propylene compressor suction drum 400 | -2-8 (e.g., 0-6 or 3) | Pv (e.g., about 500±200) |
| Between 1^{st} and 2^{nd} stage of the compressor 500 | | 600-1000 (e.g., 700-900 or 800) |
| Propylene vapor 502 | | 1300-1700 (e.g., 1400-1600 or 1500) |
| Propylene liquid 508 | | 1300-1700 (e.g., 1400-1600 or 1500) |
| Propylene liquid 514 | | 1300-1700 (e.g., 1400-1600 or 1500) |
| Superheated propylene 326 | 60-70 (e.g., 62-68 or 65) | 600-1000 (e.g., 700-900 or 800) |
| Ammonia 102 | 60-70 (e.g., 62-68 or 65) | 50-250 (e.g., 80-150 or 110) |
| Ammonia 202 | 15-25 (e.g., 18-22 or 20) | 500-2000 (e.g., 900-1500 or 1400) |
| Ammonia vapor 516 | 19-29 (e.g., 21-27 or 24) | 300-1000 (e.g., 400-600 or 500) |
| Superheated ammonia 602 | 60-70 (e.g., 62-68 or 65) | 300-700 (e.g., 400-600 or 500) |
| Hot water 118 | 25-35 (e.g., 27-33 or 30) | Pabs |
| Warm water 117 | 15-25 (e.g., 17-23 or 20) | Pabs |
| Cool water 116 | 4-13 (e.g., 6-11 or 8) | Pabs |

| | | |
|---|---|---|
| Tv is the temperature at the vapor pressure of propylene corresponding to the operating pressure. Pv is the vapor pressure of propylene at the operating temperature. P_{abs} is the operating pressure of the absorber. | | |

The open refrigerant loop and integrated feedstock delivery described above may be applied to chemical manufacturing processes that have a feed of propane or propylene and include a refrigerant duty. Examples of such chemical manufacturing processes may include propylene ammoxidation to acrylonitrile and propane dehydration to produce propylene.

For example, a propane to acrylic acid process would need a refrigeration cycle to recover the unconverted propane from the off-gas before recycling it into the reactor. This process and system for producing acrylic acid may be modified to include an open refrigerant loop and integrated feedstock delivery where the refrigerant loop includes one or more of the foregoing heat exchangers.

In another example, propane dehydration processes use a refrigeration loop to recover propylene from the cracked gas in the cold section, along with recycling unreacted propane, if any, back to the reactor. These processes and systems for producing propylene may be modified to include an open refrigerant loop and integrated feedstock delivery where the refrigerant loop includes one or more of the foregoing heat exchangers.

The foregoing description of certain examples, including illustrated examples, has been presented only for the purpose of illustration and description and is not intended to be exhaustive or to limit the disclosure to the precise forms disclosed. Numerous modifications, adaptations, and uses thereof will be apparent to those skilled in the art without departing from the scope of the disclosure. For instance, any examples described herein can be combined with any other examples to yield further examples.

## Claims

1. A chemical manufacturing process comprising:
conveying a hydrocarbon feedstock in a liquid phase from a feedstock accumulator to a shell side of at least one heat exchanger in parallel, wherein the hydrocarbon feedstock comprises propane or propylene;
vaporizing the hydrocarbon feedstock in the shell side of the at least one heat exchanger;
collecting the hydrocarbon feedstock in a vapor phase from the at least one heat exchanger in an economizer that contains the hydrocarbon feedstock in both the liquid phase and the vapor phase;
compressing and condensing a first portion of the feedstock in the vapor phase from the economizer in a compressor;
recycling the feedstock in the liquid phase from the compressor to the feedstock accumulator; and
conveying a second portion of the hydrocarbon feedstock in the vapor phase from the economizer to a reactor;
optionally, the process further comprising:
heating the second portion of the hydrocarbon feedstock from the economizer before introduction to the reactor.

2. The process of claim 1, further comprising:
letting down a pressure of the hydrocarbon feedstock in a liquid phase before introduction to the shell side of the at least one heat exchanger.

3. The process of claim 1, wherein the shell side of the at least one heat exchanger operates at a temperature ranging from 10 °C to 20 °C and at a pressure within 200 kPa of a vapor pressure of the hydrocarbon feedstock.

4. The process of claim 1, further comprising:
controlling a level of the feedstock in the liquid phase in the economizer with the feedstock in the liquid phase from the feedstock accumulator.

5. The process of claim 1, wherein the at least one heat exchanger is at least one first heat exchanger, and wherein the method further comprises:
conveying the feedstock in the liquid phase from the economizer to a shell side of at least one second heat exchanger in parallel;
vaporizing the feedstock in a shell side of the at least one second heat exchanger;
collecting the feedstock in the vapor phase from the at least one second heat exchanger in a vessel;
compressing and condensing the feedstock in the vapor phase from the vessel in a first stage of the compressor; and
wherein the compressing and condensing of the first portion of the feedstock in the vapor phase from the economizer is in a second stage of the compressor; optionally, the process further comprising:
letting down a pressure of the hydrocarbon feedstock in a liquid phase before introduction to the shell side of the at least one second heat exchanger; or
wherein the shell side of the at least one heat second exchanger operates at a temperature ranging from -2 °C to 8 °C and at a pressure within 200 kPa of a vapor pressure of the hydrocarbon feedstock.

6. The process of claim 1, wherein the at least one heat exchanger is at least one first heat exchanger, and wherein the method further comprises: passing the feedstock in the liquid phase from the compressor through a tube side of at least one second heat exchanger in parallel before introduction to the feedstock accumulator.

7. The process of claim 6, wherein the reactor is an ammoxidation reactor, and wherein the method further comprises: passing an ammonia feedstock through a shell side of one or more of the at least one second heat exchanger; optionally, wherein the ammonia feedstock after passing through the shell side of the one or more of the at least one second heat exchanger is at a temperature ranging from 19 °C to 29 °C; or the process further comprising: heating the ammonia feedstock downstream of the one or more of the at least one second heat exchanger to a temperature ranging from 60 °C to 70 °C before introduction to the reactor.

8. A chemical manufacturing system comprising:
a reactor;
at least one heat exchanger in parallel;
a feedstock accumulator;
an economizer; and
a compressor,
wherein the chemical manufacturing system includes:
an open refrigerant cycle flow path comprising, in a flow direction of a hydrocarbon feedstock: the feedstock accumulator, a shell side of the at least one heat exchanger, the economizer, the compressor, and the feedstock accumulator, and
a feedstock delivery flow path comprising, in a flow direction of the hydrocarbon feedstock: the feedstock accumulator, the shell side of the at least one heat exchanger, the economizer, and the reactor.

9. The chemical manufacturing system of claim 8, wherein the at least one heat exchanger is at least one first heat exchanger, wherein the open refrigerant cycle flow path further comprises a shell side of at least one second heat exchanger in parallel and a vessel in series and between the economizer and a first stage of the compressor, and wherein the open refrigerant cycle flow path further comprises a second stage of the compressor immediately downstream of the economizer.

10. The chemical manufacturing system of claim 9, wherein the open refrigerant cycle flow path further comprises at least one control valve between the economizer and the shell side of the at least one second heat exchanger.

11. The chemical manufacturing system of claim 8, wherein the at least one heat exchanger is at least one first heat exchanger, and wherein the open refrigerant cycle flow path further comprises a tube side of at least one second heat exchanger in parallel between the compressor and the feedstock accumulator.

12. The chemical manufacturing system of claim 11, further comprising an ammonia feedstock flow path to the reactor that passes through a shell side of one or more of the at least one second compressor before introduction to the reactor.

13. The chemical manufacturing system of claim 8, wherein the open refrigerant cycle flow path further comprises at least one control valve between the feedstock accumulator and the shell side of the at least one heat exchanger.

14. An ammoxidation process comprising:
reacting propylene, ammonia, and air in a reactor to yield a raw product;
contacting the raw product with water in an absorber;
passing water extracted from the absorber through a tube side of at least one heat exchanger in parallel in a water cooling cycle, wherein the water cooling cycle cools the water extracted from the absorber and recycles the water extracted from the absorber back to the absorber; and
passing the propylene through a shell side of the at least one heat exchanger before introducing the propylene to the reactor.

15. The ammoxidation process of claim 14, wherein the at least one heat exchanger is at least one first heat exchanger, and wherein the method further comprises: passing the water extracted from the absorber through a tube side of at least one second heat exchanger in parallel that is downstream of the at least one first heat exchanger.
